# EUROPEAN PATENT SPECIFICATION

(11) **EP 4 626 850 B1**
(45) Date of publication and mention of the grant of the patent: **22.07.2026**
(21) Application number: 23817972.5
(22) Date of filing: 01.12.2023
(51) Int. Cl.: C07C 1/24, C07C 29/141, C07C 41/26, C07C 45/51, C07D 309/04

(54) **PROPYLENE POLYOL CONVERSION TO OLEFIN MONOMER**
UMWANDLUNG VON PROPYLEN IN OLEFINMONOMER
CONVERSION DE PROPYLÈNE POLYOL EN MONOMÈRE OLÉFINIQUE

(30) Priority: 02.12.2022 US 202263385875 P
(43) Date of publication of application: 08.10.2025
(73) Proprietor: Basell Poliolefine Italia S.r.l., 20121 Milano (IT)
(72) Inventor: NAGY, Sandor, Houston, Texas 77010 (US); MANNEL, David S., Houston, Texas 77010 (US); NAGY, Natalia, Houston, Texas 77010 (US); ROSE, Amy D., Houston, Texas 77010 (US); HORVAT, George R., Houston, Texas 77010 (US); ARNDT, Larry W., Houston, Texas 77010 (US); LEYSHON, David W., Houston, Texas 77010 (US); YANG, Xueyong, Houston, Texas 77010 (US); KIMMICH, Barbara, Houston, Texas 77010 (US)
(74) Representative: LyondellBasell
(86) International application number: PCT/EP2023/083859
(87) International publication number: WO 2024/115715

(56) References cited:
- AI SHUO ET AL: "Kinetic study on catalytic dehydration of 1,2-propanediol and 1,2-butanediol over H-Beta for bio-ethylene glycol purification", CHEMICAL ENGENEERING JOURNAL, vol. 335, 1 March 2018 (2018-03-01), AMSTERDAM, NL, pages 530 - 538, XP093136274, ISSN: 1385-8947, Retrieved from the Internet <URL:https://www.sciencedirect.com/science/article/pii/S1385894717318491/pdfft?md5=dffec5802d438a4080bc6a0637f64def&pid=1-s2.0-S1385894717318491-main.pdf> DOI: 10.1016/j.cej.2017.10.134
- OTOMO RYOICHI ET AL: "Selective Dehydration of 1,2-Propanediol to Propanal over Boron Phosphate Catalyst in the Presence of Steam", ACS SUSTAINABLE CHEMISTRY & ENGINEERING, vol. 7, no. 3, 4 February 2019 (2019-02-04), US, pages 3027 - 3033, XP093136357, ISSN: 2168-0485, DOI: 10.1021/acssuschemeng.8b04594
- DAZHI ZHANG ET AL: "Dehydration of 1,2-propanediol to propionaldehyde over zeolite catalysts", APPLIED CATALYSIS A: GENERAL, ELSEVIER, AMSTERDAM, NL, vol. 400, no. 1, 21 April 2011 (2011-04-21), pages 148 - 155, XP028228388, ISSN: 0926-860X, [retrieved on 20110428], DOI: 10.1016/J.APCATA.2011.04.028
- HACATRJAN SCHANTH ET AL: "Titania-supported molybdenum oxide combined with Au nanoparticles as a hydrogen-driven deoxydehydration catalyst of diol compounds", CATALYSIS SCIENCE & TECHNOLOGY, vol. 12, no. 7, 4 April 2022 (2022-04-04), UK, pages 2146 - 2161, XP093136443, ISSN: 2044-4753, DOI: 10.1039/D1CY02144C
- GONGMING PENG ET AL: "Zirconia-supported niobia catalyzed formation of propanol from 1,2-propanediol via dehydration and consecutive hydrogen transfer", JOURNAL OF INDUSTRIAL AND ENGINEERING CHEMISTRY, vol. 20, no. 5, 1 September 2014 (2014-09-01), KOREA, pages 2641 - 2645, XP055242685, ISSN: 1226-086X, DOI: 10.1016/j.jiec.2013.11.042

## Description

### CROSS-REFERENCE TO RELATED APPLICATIONS

The application is filed under the Patent Cooperation Treaty, which claims the benefit of priority to U.S. Provisional Patent Application No. 63/385,875, filed on December 2, 2022.

### FIELD OF THE DISCLOSURE

The disclosure relates to methods of catalytically converting propylene glycols into propylene and/or propylene precursors. Methods for conversion of propylene glycols (or intermediate products) are successively reacted with a dehydration cleavage catalyst, a hydrogenation catalyst, and optionally a dehydration catalyst.

### BACKGROUND OF THE DISCLOSURE

Propylene glycol substances are found in waste streams and/or by-products associated with a number of industrial processes. Such waste streams and by-products can include a number other constituents and in some cases a significant amount of water. Significant sources of propylene glycols occur during recycling of certain post-consumer waste oxygen-containing plastics, are produced as waste streams during resulting from the production of propylene oxides, and are a by-product of aircraft de-icing and other industrial activities where products containing propylene glycol are used as an anti-freeze agent. Such propylene glycol substances are miscible in water and exhibit a low potential to volatilize from water or soil in both pure and dissolved forms. Propylene glycol exerts high levels of biochemical oxygen demand during degradation in surface waters.

Heightened standards of living and increased urbanization have led to an increased demand for polymer products, including, but not limited to, polyurethane ("PU") and polyethylene terephthalate ("PET"). PET is the most common thermoplastic polymer resin of the polyester family and is used in fibers for clothing, containers for liquids and foods, thermoforming for manufacturing, and in combination with glass fiber for engineering resins. The downside to the demand for polymer products is the increase in waste. Post-consumer plastic waste typically ends up in landfills, with about 12% being incinerated and about 9% being diverted to recycling. In landfills, most plastics do not degrade quickly, becoming a major source of waste that overburdens the landfill. Incineration is also not an ideal solution to treating the plastic wastes as incineration leads to the formation of carbon dioxide and other greenhouse gas emissions. As such, there has been much interest in developing methods of recycling plastic waste to reduce the burden on landfills while being environmentally friendly. Post-consumer waste comprising PU and PET can be degraded by a glycolysis reaction with propylene glycols in the presence of trans-esterification catalysts, resulting in a glycolic component comprising propylene glycols (mono-, di-, and/or higher propylene glycols), which may further include butane-diol, ethylene glycols, and other oxygenated components.

One process for production of propylene oxide, known as PO/TBA, involves the co-oxidation of isobutylene, producing propylene oxide and tertiary butyl alcohol. Another process for production of propylene oxide, known as POSM, involves the co-oxidation of ethyl benzene, producing propylene oxide and styrene monomer. These processes produce waste streams comprising propylene glycols (mono-, di-, and/or higher propylene glycols.

Deicing fluids come in a variety of types and are typically composed of ethylene glycol (EG) or propylene glycol (PG), along with other ingredients. Propylene glycol-based fluid is more common because it is less toxic than ethylene glycol. Deicing a large commercial aircraft produces significant amounts of diluted propylene glycol waste fluid. Propylene glycol is also used as an anti-freeze fluid in geothermal wells where leakage of propylene glycol can affect dissolved oxygen on ground and surface water.

There is a continued need for the development of a robust processes for the conversion of propylene glycols to higher value and/or environmentally desirable dispositions. Ideally, such processes would be highly flexible and could be implemented with commonly used equipment and familiar techniques to produce a wide variety of products. Ai Shuo et al, Chemical Engeneering Journal, vol. 335, p. 530-538 discloses the dehydration of 1,2-propylene glycol using H-beta zeolite. Dipropylene glycol and tripropylene glycol are formed during the reaction but are later consumed. The main products are 2-ethyl-4-methyl-1,3-dioxalane and propanal. Propanal is also consumed in the course of the reaction.

### SUMMARY OF THE DISCLOSURE

The present disclosure provides methods for catalytic conversion of propylene polyols to higher value products useful as feedstocks to other processes. In some embodiments, a process comprises adding a feed stream comprising one or more propylene polyols, and optionally water, to a dehydration cleavage reaction zone in the presence of a dehydration cleavage catalyst to form a first reaction mixture. The first reaction mixture is reacted under temperature and pressure conditions sufficient to produce a dehydration cleavage product stream comprising propionaldehyde, a dioxolane component, and a dioxane component. In some embodiments, at least a portion of the dehydration cleavage product stream is recycled as a portion of the feed to the dehydration cleavage reaction zone.

The dehydration cleavage product stream can be fed along with hydrogen to a hydrogenation reaction zone in the presence of a hydrogenation catalyst to form a second reaction mixture. Alternatively, the dehydration cleavage product stream can be sent to a first distillation column to produce a dehydration cleavage product overhead stream and a dehydration cleavage product bottoms stream. The first distillation column is a fractioning or distillation column and includes equipment associated with the column, such as heat exchangers, decanters, pumps, compressors, valves, and the like. The dehydration cleavage product overhead stream can be fed along with hydrogen to the hydrogenation reaction zone in the presence of a hydrogenation catalyst to form an alternate second reaction mixture.

The second reaction mixture or the alternate second reaction mixture is reacted under temperature and pressure conditions sufficient to produce a hydrogenation product stream comprising a propanol component. In some embodiments, the hydrogenation product stream can be fed to a dehydration reaction zone in the presence of a dehydration catalyst to form a third reaction mixture. Alternatively, in some embodiments, the hydrogenation product stream can be sent to a second distillation column to produce a hydrogenation product overhead stream and a hydrogenation product bottoms stream. The second distillation column is a fractioning or distillation column and includes equipment associated with the column, such as heat exchangers, decanters, pumps, compressors, valves, and the like. The hydrogenation product overhead stream can be fed to the dehydration reaction zone in the presence of a dehydration catalyst to form an alternate third reaction mixture.

In some embodiments, the third reaction mixture or the alternate third reaction mixture is reacted under temperature and pressure conditions sufficient to produce a dehydration product stream comprising propylene for further processing. Alternatively, in some embodiments, the dehydration product stream can be sent to a third distillation column to produce a dehydration product overhead stream comprising propylene and a dehydration product bottoms stream comprising water. The third distillation column is a fractioning or distillation column and includes equipment associated with the column, such as heat exchangers, decanters, pumps, compressors, valves, and the like.

In some embodiments, the process further comprises adding an organic waste stream, comprising one or more propylene polyols and a first content of one or more impurities harmful to the dehydration cleavage catalyst, to a guard reaction zone to form the feed stream comprising one or more propylene polyols.

The foregoing has outlined rather broadly the features and technical advantages of the present disclosure in order that the detailed description of the disclosure that follows may be better understood. Additional features and advantages of the disclosure will be described hereinafter, which form the subject matter of the claims of the disclosure. It should be appreciated by those skilled in the art that the conception and specific embodiments disclosed may be readily utilized as a basis for modifying or designing other catalyst compositions and/or processes for carrying out the same purposes of the present disclosure. The novel features which are believed to be characteristic of the disclosure, both as to its compositions and methods, together with further objects and advantages will be better understood from the following description.

### BRIEF DESCRIPTION OF THE FIGURES

The claimed subject matter may be understood by reference to the following description taken in conjunction with the accompanying drawings, in which like reference numerals identify like elements, and in which:
**FIG. 1A** is a simplified flow diagram of the disclosed process having a dehydration cleavage reaction zone and a hydrogenation reaction zone, according to embodiments of the disclosure;
**FIG. 1B** is a simplified flow diagram of the dehydration reaction zone, optionally used in conjunction with the flow shown in **FIG. 1A****,** according to embodiments of the disclosure;
**FIG. 1C** is a simplified flow diagram of the guard reaction zone, optionally used in conjunction with the flow shown in **FIG. 1A** or the combination of **FIG. 1A** and **FIG 1B****,** according to embodiments of the disclosure;
**FIG. 2A** is a simplified flow diagram of the disclosed process having a dehydration cleavage reaction zone followed by a first distillation column and a hydrogenation reaction zone followed by a second distillation column, according to embodiments of the disclosure;
**FIG. 2B** is a simplified flow diagram of the dehydration reaction zone followed by a third distillation column, optionally used in conjunction with the flow shown in **FIG. 2A****,** according to embodiments of the disclosure; and
**FIG. 2C** is a simplified flow diagram of the guard reaction zone, optionally used in conjunction with the flow shown in **FIG. 2A** or the combination of **FIG. 2A** and **FIG 2B****,** according to embodiments of the disclosure.

While the disclosed process and composition are susceptible to various modifications and alternative forms, the drawings illustrate specific embodiments herein described in detail by way of example.

### DETAILED DESCRIPTION OF THE DISCLOSURE

Illustrative embodiments of the subject matter claimed below will now be disclosed. In the interest of clarity, some features of some actual implementations may not be described in this specification. It will be appreciated that in the development of any such actual embodiments, numerous implementation-specific decisions must be made to achieve the developer's specific goals, such as compliance with system-related and business-related constraints, which will vary from one implementation to another. Moreover, it will be appreciated that such a development effort, even if complex and time-consuming, would be a routine undertaking for those of ordinary skill in the art having the benefit of this disclosure.

The words and phrases used herein should be understood and interpreted to have a meaning consistent with the understanding of those words and phrases by those skilled in the relevant art. No special definition of a term or phrase, i.e., a definition that is different from the ordinary and customary meaning as understood by those skilled in the art, is intended to be implied by consistent usage of the term or phrase herein. To the extent that a term or phrase is intended to have a special meaning, i.e., a meaning other than the broadest meaning understood by skilled artisans, such a special or clarifying definition will be expressly set forth in the specification in a definitional manner that provides the special or clarifying definition for the term or phrase. It must also be noted that, as used in the specification and the appended claims, the singular forms "a," "an," and "the" include plural references unless otherwise specified.

For example, the following discussion contains a non-exhaustive list of definitions of several specific terms used in this disclosure (other terms may be defined or clarified in a definitional manner elsewhere herein). These definitions are intended to clarify the meanings of the terms used herein. It is believed that the terms are used in a manner consistent with their ordinary meaning, but the definitions are nonetheless specified here for clarity.

### Definitions

As used herein, "propylene glycol," in reference to the feed stream to the process disclosed herein, refers to mono-propylene glycol, di-propylene glycol, tri-propylene glycol, tetra-propylene glycol, higher polypropylene glycols, or a combination thereof.

As used herein, "impurities," in reference to the organic waste stream, refers to material present that can reduce the abilities of a solid acid catalyst to perform dehydration cleavage of the propylene glycols in the feed to the dehydration cleavage reaction zone. In some embodiments, impurities comprise amines, urethane, amides, other nitrogen containing hydrocarbons, organic bases, caustic, or a combination thereof.

As used herein, "post-consumer waste" refers to a type of waste produced by the end consumer of a material stream.

As used herein, "post-industrial waste" refers to a type of waste produced during the production process of a product.

As used herein, "reaction zone" refers to a chamber sufficiently enclosed to maintain selected operating conditions within the chamber to produce a desired reaction, such as a dehydration cleavage reaction zone, a hydrogenation reaction zone, or a dehydration reaction zone. In some embodiments, each reaction zone can be a separate reactor. In some embodiments, a single vessel can contain a plurality of reaction zones.

As used herein, "waste stream" is a type of feed stream comprising material that has been discarded as no longer useful, including but not limited to, post-consumer and post-industrial waste.

As used herein, "zeolite" refers to an aluminosilicate mineral with a microporous structure. Zeolites are, in one aspect, useful as catalysts for the processes disclosed herein. Zeolites can occur naturally or can be produced industrially.

It is noted that in this disclosure and particularly in the claims and/or paragraphs, terms such as "comprises", "comprised", "comprising" and the like can have the meaning attributed to it in patent law; e.g., they can mean "includes", "included", "including", and the like; and that terms such as "consisting essentially of" and "consists essentially of" have the meaning ascribed to them in U.S. patent law, e.g., they allow for elements not explicitly recited, but exclude elements that are found in the prior art or that affect a basic or novel characteristic of the disclosure.

The term "pure" as used in reference to the feed stream refers to a feed that is 100% polyolefin, but does not mean that the feed contains only one type of polyolefin. Rather, a "pure" feed stream can have a mixture of polyolefins such as low-density polyethylene, high density polyethylene, polypropylene and combinations thereof.

All concentrations herein are by weight percent ("wt. %") unless otherwise specified.

The use of the word "a" or "an" when used in conjunction with the term "comprising" in the claims or the specification means one or more than one, unless the context dictates otherwise.

The term "about" means the stated value plus or minus the margin of error of measurement or plus or minus 10% if no method of measurement is indicated.

The use of the term "or" in the claims is used to mean "and/or" unless explicitly indicated to refer to alternatives only or if the alternatives are mutually exclusive.

The terms "comprise", "have", "include" and "contain" (and their variants) are openended linking verbs and allow the addition of other elements when used in a claim.

The phrase "consisting of" is closed and excludes all additional elements.

The phrase "consisting essentially of" excludes additional material elements but allows the inclusions of non-material elements that do not substantially change the nature of the disclosure.

The phrase "substantially all of" means greater than or equal to 95 wt%, greater than or equal to 99 wt%, greater than or equal to 99.5 wt%, or greater than or equal to 99.9 wt%.

The following abbreviations are used herein:

| ABBREVIATION | TERM |
|---|---|
| Beta | Beta (or BEA) zeolite |
| DPG | Di-propylene glycol |
| H-USY | H-ultra-stable zeolite Y |
| MPG | Mono-propylene glycol |
| PET | poly(ethylene terephthalate) |
| PPO | Propylene polyol |
| PU | Polyurethane |
| T4PG | Tetra-propylene glycol |
| TGA | Thermogravimetric Gravimetric Analysis |
| TPG | Tri-propylene glycol |
| USY | Ultra-stable zeolite Y |
| wt% | Weight percent |
| WHSV | Weight hourly space velocity |
| ZSM-5 | Zeolite Socony Mobil-5 |

### Catalytic conversion of propylene polyols

The present disclosure provides catalytic conversion of propylene polyols to produce products comprising propylene and/or propylene precursors. In some embodiments, a process comprised adding a feed stream comprising one or more propylene glycols to a dehydration cleavage reaction zone comprising a dehydration cleavage catalyst to form a first reaction mixture. The first reaction mixture is reacted under temperature and pressure conditions sufficient to form a dehydration cleavage product comprising propionaldehyde, a dioxolane component, and a dioxane component. In some embodiments, at least a portion of the dehydration cleavage product is recycled as a portion of the feed to the dehydration cleavage reaction zone.

In some embodiments, the dehydration cleavage product is sent as a feed stream to a hydrogenation reaction zone. Alternatively, the dehydration cleavage product is sent to a first distillation column to form a dehydration cleavage product overhead stream comprising propionaldehyde and a dehydration cleavage product bottoms stream, and the dehydration cleavage product overhead stream is sent as a feed stream to a hydrogenation reaction zone. In the alternative embodiments where the dehydration cleavage product bottoms stream is formed, at least a portion of the dehydration cleavage product bottoms stream is recycled as additional feed to the dehydration cleavage reaction zone.

The dehydration cleavage product, or alternatively the dehydration cleavage product overhead stream, and hydrogen are added to a hydrogenation zone comprising a hydrogenation catalyst to form a second reaction mixture. The second reaction mixture is reacted under temperature and pressure conditions sufficient to form a hydrogenation product comprising a propanol component. In some embodiments, at least a portion of the hydrogenation product is recycled as a portion of the feed to the dehydration cleavage reaction zone, the hydrogenation reaction zone, or a combination thereof.

In some embodiments, the hydrogenation product is sent as a feed stream to a dehydration reaction zone. Alternatively, the hydrogenation product is sent to a second distillation column to form a hydrogenation product overhead stream comprising a propanol component and a hydrogenation product bottoms stream, and the hydrogenation product overhead stream is sent as a feed stream to a dehydration reaction zone.

The hydrogenation product, or alternatively the hydrogenation product overhead stream, are added to a dehydration zone comprising a dehydration catalyst to form a third reaction mixture. In the alternative embodiments where the hydrogenation product bottoms stream is formed, at least a portion of the hydrogenation product bottoms stream is recycled as additional feed to the dehydration cleavage reaction zone, the hydrogenation reaction zone, or a combination thereof. The third reaction mixture is reacted under temperature and pressure conditions sufficient to form a dehydration product comprising propylene.

In some embodiments, the dehydration product is sent to further processing for recovery of the propylene. Alternatively, the dehydration product is sent to a third distillation column to form a dehydration product overhead stream comprising propylene and a dehydration product bottoms stream comprising water, and the dehydration product overhead stream is sent to further processing for recovery of the propylene.

In some embodiments, the process further comprises adding an organic waste stream, comprising one or more propylene polyols and a first content of one or more impurities harmful to the dehydration cleavage catalyst, to a guard reaction zone to form the feed stream comprising one or more propylene polyols. In some embodiments, such impurities comprise amines, urethane, amides, other nitrogen containing hydrocarbons, organic bases, caustic, or a combination thereof. In some embodiments, the guard reaction zone comprises a reactive bed comprising generic absorbants, clays, diatomites, activated carbon, or a combination thereof. In some embodiments, the guard reaction zone can be operated at a pressure in the range of from 0 psig (0 kPag) to 10 psig (69 kPag) and a temperature in the range of from 20°C to 30°C.

### Dehydration cleavage reaction

The feed to the dehydration cleavage reaction zone comprises one or more propylene polyols and optionally water. The dehydration cleavage reaction zone comprises a dehydration cleavage catalyst. In some embodiments, the one or more propylene polyols comprise propylene glycol, di-propylene glycol, tri-propylene glycol, tetra-propylene glycol, or a combination thereof. A first reaction mixture is formed from the feed and the catalyst when the feed is added to the reaction zone.

In some embodiments, the first reaction mixture is reacted at: a temperature in the range of from 20°C to 600°C, from 50°C to 500°C, or from 100°C to 300°C; a pressure in the range of from 0 psig (0 kPag) to 1,000 psig (6,895 kPag), from 10 psig (68.9 kPag) to 300 psig (2,068 kPag), or from 50 psig (345 kPag) to 100 psig (689 kPag); or a combination thereof.

In some embodiments, the one or more polyols and optionally water are added to the dehydration cleavage reaction zone at a weight hourly space velocity in the range of from 0.1 h⁻¹ to 100 h⁻¹, 0.5 h⁻¹ to 60 h⁻¹, or 0.8 h⁻¹ to 25 h⁻¹.

In some embodiments, the reaction produces a dehydration cleavage product stream comprising propionaldehyde, a dioxolane component, a dioxane component, 1-propanol, MPG, DPG, and other oxygenates (0-30%). In some embodiments, the dehydration cleavage product stream comprises water in the range of from 0 wt% to 90 wt%, wherein weight percentage water is based on the total weight of the dehydration cleavage product, and the propionaldehyde in the range of from 40 wt% to 80 wt%, the dioxolane component in the range of 5 wt% to 50 wt%, the dioxane component in the range of from 1 wt% to 20 wt%, the 1-propanol in the range of 0 wt% to 10 wt%, the MPG in the range of 0 wt% to 10 wt%, the DPG in the range of 0 wt% to 10 wt%, and the other oxygenates in the range of 0 wt% to 30 wt%, wherein weight percentages are based on the total weight of the dehydration cleavage product stream other than water.

In some embodiments, the dehydration cleavage product stream is fed to a first distillation column to produce a dehydration cleavage product overhead stream comprising propionaldehyde and a dehydration cleavage product bottoms stream. In some embodiments, the dehydration cleavage product overhead stream is fed to a hydrogenation reaction zone, the dehydration cleavage product bottoms stream is recycled as additional feed to the dehydration cleavage reaction zone.

### Dehydration cleavage catalyst

In some embodiments, the dehydration cleavage catalyst comprises a first solid acid catalyst component. In some embodiments, the solid acid catalyst component comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, one or more polystyrene sulfonate acidic resins, sulfonate functionalized support, tethered organic sulfonic acids, acidic clays, or a combination thereof.

In some embodiments, the first solid acid catalyst is further characterized by one or more of the following:
a) the zeolite component comprises zeolite-H-Y, zeolite-H-ZSM5, zeolite-H-beta, zeolite-H-mordenite, zeolite-H-ferrierite, or a combination thereof;
b) the alumina silicate comprises amorphous alumina silicate, acid washed layered alumina silicate, or a combination thereof, wherein in some embodiments, the acid washed layered alumina silicate comprises bentonite, vermiculite, or a combination thereof;
c) the aluminum phosphate comprises SAPO 34;
d) the supported phosphoric acid comprises a silica, clay, or alumina support;
e) the one or more supported tungsten oxides comprise a silica, clay, or alumina support;
f) the one or more tungsten oxides comprise tungsten oxide, tungsten di-oxide, tungsten tri-oxide, tungsten pentoxide, or a combination thereof;
g) the supported tungstosilicic acid comprises a silica, clay, or alumina support; the supported phosphomolybdic acid comprises a silica, clay, or alumina support; and the sulfonate functionalized support is silica.

### Hydrogenation reaction

In some embodiments, the feed to the hydrogenation reaction zone comprises the dehydration cleavage product. In some embodiments, the dehydration cleavage product is sent to a distillation column to produce a dehydration cleavage product overhead stream and a dehydration cleavage product bottoms stream, and the dehydration cleavage product overhead stream is sent as feed to the hydrogenation reaction zone. A second reaction mixture is formed from the feed, hydrogen, and the catalyst when the feed and hydrogen are added to the hydrogenation reaction zone. In some embodiments, the hydrogen is added in an amount such that the molar ratio of hydrogen (H₂) to oxygen (O) in the feed is in the range of from 1 to 20 or from 5 to 10.

In some embodiments, the second reaction mixture is reacted at: a temperature in the range of from 20°C to 600°C, from 50°C to 500°C, or from 100°C to 350°C; a pressure in the range of from 100 psig (689 kPag) to 1,500 psig (10,340 kPag), 250 psig (1,724 kPag) to 1,250 psig (8,620 kPag), or from 500 psig (3,450 kPag) to 1,000 psig (6,890 kPag); or a combination thereof.

In some embodiments, the dehydration cleavage product stream and hydrogen are added to the hydrogenation reaction zone at a weight hourly space velocity in the range of from 0.1 h⁻¹ to 100 h⁻¹, 0.3 h⁻¹ to 50 h⁻¹, or 0.5 h⁻¹ to 5 h⁻¹; or any combination thereof.

In some embodiments, the feed stream is added to the dehydration cleavage reaction zone at a weight hourly space velocity in the range of from 0.1 h⁻¹ to 100 h⁻¹, 0.5 h⁻¹ to 60 h⁻¹, or 0.8 h⁻¹ to 25 h⁻¹; the dehydration cleavage product stream and hydrogen are added to the hydrogenation reaction zone at a weight hourly space velocity in the range of from 0.1 h⁻¹ to 100 h⁻¹, 0.3 h⁻¹ to 50 h⁻¹, or 0.5 h⁻¹ to 5 h⁻¹; or any combination thereof.

The reaction produces a hydrogenation product stream comprising a propanol component. In some embodiments, the propanol component comprises n-propanol, iso-propanol, or a combination thereof. In some embodiments, the hydrogenation product stream comprises 0 wt% to 90 wt% water and 10 wt% to 100 wt% organics other than water. In some embodiments, the organics other than water comprise 1-propanol in the range of from 50 wt% to 90 wt% and other C₃ hydrocarbons in the range of from 10 wt% to 50 wt%, wherein weight percentages are based on the total weight of the organics other than water.

In some embodiments, the hydrogenation product stream is fed to a dehydration reaction zone. In some embodiments, the hydrogenation product stream is fed to a second distillation column to produce a hydrogenation product overhead stream comprising the propanol component and a hydrogenation product bottoms stream. In some embodiments, the hydrogenation product overhead stream is fed to a dehydration reaction zone, the hydrogenation product bottoms stream is recycled as additional feed to the hydrogenation reaction zone, the dehydration cleavage reaction zone, or a combination thereof.

### Hydrogenation catalyst

Any one of the foregoing embodiments can be further characterized in that the hydrogenation catalyst comprises nickel (Ni), Raney Ni, cobalt (Co), molybdenum (Mo), ceria (Ce), magnesium (Mg), gold (Au), iridium (Ir), osmium (Os), palladium (Pd), platinum (Pt), rhodium (Rh), ruthenium (Ru), tungsten (W), titanium (Ti), NiMo, CoMo, NiW, CoW, Ru, Pt, Pd, or a combination thereof. In some embodiments, the foregoing metal or metals are supported on silica, alumina, silica alumina, zeolite, activated carbon, or a combination thereof. In some embodiments, the catalyst is sulfided prior to hydrogenation. In some embodiments, the hydrogenation catalyst comprises sulfided NiMo/Al₂O₃, sulfided CoMo/Al₂O₃, Ni/SiO₂, Ni/Al₂O₃, Raney Ni, Cu/SiO₂, Cu/Al₂O₃, Pd/SiO₂, Pd/Al₂O₃, Pd/C, Pt/SiO₂, Pt/Al₂O₃, Ru/C, In₂O₃ In₂O₃/Al₂O₃, In₂O₃/SiO₂, or a combination thereof.

### Dehydration reaction

In some embodiments, the feed to the dehydration reaction zone comprises the hydrogenation product. In some embodiments, the hydrogenation product is sent to a distillation column to produce a hydrogenation product overhead stream and a hydrogenation product bottoms stream, and the hydrogenation product overhead stream is sent as feed to the hydrogenation reaction zone. A third reaction mixture is formed from the feed, and the catalyst when the feed are added to the hydrogenation reaction zone.

In some embodiments, the third reaction mixture is reacted at: a temperature in the range of from 20°C to 600°C, from 50°C to 400°C, or from 100°C to 300°C; a pressure in the range of from 15 psig (103 kPag) to 500 psig (3,447 kPag), from 100 psig (689 kPag) to 450 psig (3,100 kPag), or from 200 psig (1,379 kPag) to 400 psig (2,760 kPag); or a combination thereof.

In some embodiments, the hydrogenation product stream or the hydrogenation product overhead stream is added to the dehydration reaction zone at a weight hourly space velocity in the range of from 0.1 h⁻¹ to 100 h⁻¹, 0.5 h⁻¹ to 60 h⁻¹, or 0.8 h⁻¹ to 25 h⁻¹.

The reaction produces a dehydration product stream comprising propylene. In some embodiments, the dehydration product stream comprises propylene in the range of from 80 wt% to 100 wt% and other hydrocarbons in the range of from 0 wt% to 20 wt%, wherein weight percentages are based on the total weight of the dehydration product stream. In some embodiments, the other hydrocarbons comprise C₂, C₃, and/or C₄ oxygenates, C₂, C₃, and/or C₄ olefins, or a combination thereof.

In some embodiments, the dehydration product stream is sent to further processing and recovery of the propylene. In some embodiments, the dehydration product stream is fed to a third distillation column to produce a dehydration product overhead stream comprising the propylene and a dehydration product bottoms stream. In some embodiments, the dehydration product overhead stream is sent to further processing and recovery of the propylene.

### Dehydration catalyst

In some embodiments, the dehydration catalyst comprises a second solid acid catalyst component. In some embodiments, the second solid catalyst component comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, or a combination thereof.

In some embodiments, the second solid acid catalyst is further characterized by one or more of the following:
a) the zeolite component comprises zeolite-H-Y, zeolite-H-ZSM5, zeolite-H-beta, zeolite-H-mordenite, zeolite-H-ferrierite, or a combination thereof;
b) the alumina silicate comprises amorphous alumina silicate, acid washed layered alumina silicate, or a combination thereof, wherein the acid washed layered alumina silicate comprises bentonite, vermiculite, or a combination thereof;
c) the aluminum phosphate comprises SAPO 34;
d) the supported phosphoric acid comprises a silica, clay, or alumina support;
e) the one or more supported tungsten oxides comprise a silica, clay, or alumina support; and
f) the one or more tungsten oxides comprise tungsten oxide, tungsten di-oxide, tungsten tri-oxide, tungsten pentoxide, or a combination thereof.

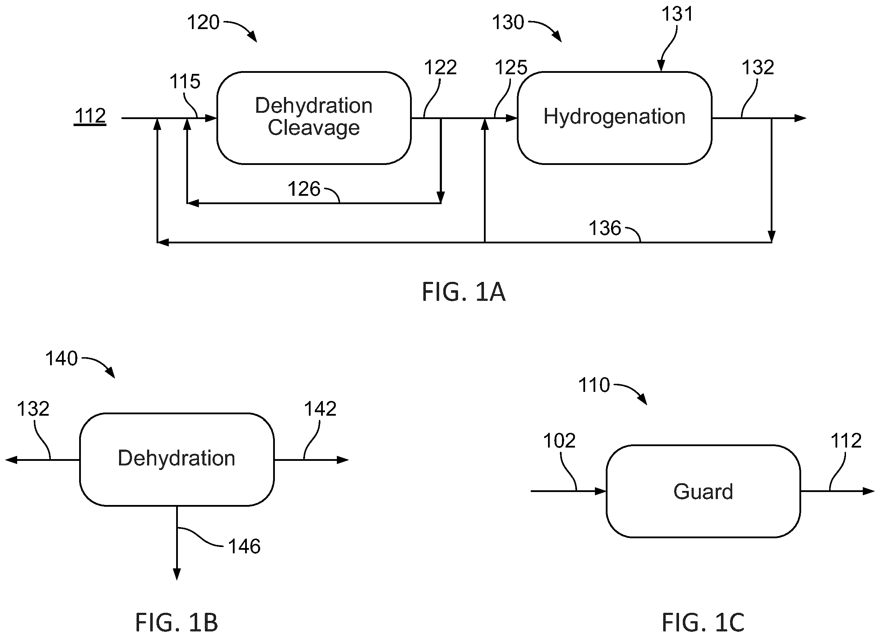

**FIG. 1A****-****FIG. 1C** show embodiments without a distillation column after each reaction zone. In **FIG. 1A****,** a feed comprising one or more propylene glycols **112** is obtained as feed to dehydration cleavage reaction zone **120.** In some embodiments, streams **126** and a portion **136** are added with stream **112** to form the feed stream to the dehydration cleavage reaction zone **120** comprising a dehydration cleavage catalyst. These streams can be mixed prior to addition to dehydration cleavage reaction zone **120** or alternatively added to dehydration cleavage reaction zone **120** at different locations.

After the reaction in dehydration cleavage reaction zone **120,** dehydration cleavage product **122** is withdrawn from dehydration cleavage reaction zone **120** and added as a feed stream to hydrogenation reaction zone **130** along with hydrogen **132.** In some embodiments, a portion of dehydration cleavage product **122** is sent as recycle stream **126** as additional feed to dehydration cleavage reaction zone **120.** In some embodiments, a portion of stream **136** is added with stream **122** to form the feed stream **125** to the hydrogenation reaction zone **130** comprising a hydrogenation catalyst. After the reaction in hydrogenation reaction zone **130,** hydrogenation product **132** is withdrawn from hydrogenation reaction zone **130.** In some embodiments, a portion of stream **132** is recycled as stream **136** and sent as additional feed to the hydrogenation reaction zone **130,** the dehydration cleavage reaction zone **120,** or a combination thereof.

In some embodiments, as shown in **FIG. 1B****,** hydrogenation product **132** is added as a feed stream to dehydration reaction zone **140.** After the reaction in dehydration reaction zone **140,** dehydration product **142** is withdrawn from dehydration reaction zone **140.** Water **146** is also withdrawn from dehydration reaction zone **140** as a by-product of the dehydration reaction.

In some embodiments, as shown in **FIG. 1C****,** an organic waste stream **102** containing impurities harmful to the dehydration cleavage catalyst is added as a feed stream **102** to guard reaction zone **110.** Guard reaction product **112** comprising on or more propylene glycols is withdrawn from guard reaction zone **110.**

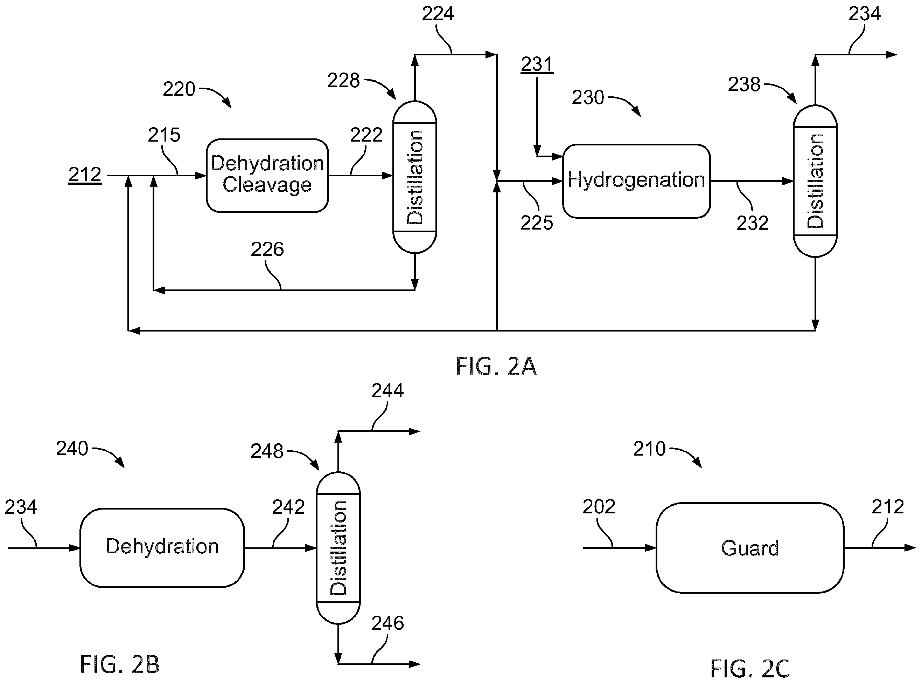

**FIG. 2A****-****FIG. 2C** show embodiments with a distillation column after each reaction zone. In **FIG. 2A****,** a feed comprising one or more propylene glycols **212** is obtained as feed to dehydration cleavage reaction zone **220.** In some embodiments, streams **226** and a portion **236** are added with stream **212** to form the feed stream to the dehydration cleavage reaction zone **220** comprising a dehydration cleavage catalyst. These streams can be mixed prior to addition to dehydration cleavage reaction zone **220** or alternatively added to dehydration cleavage reaction zone **220** at different locations.

After the reaction in dehydration cleavage reaction zone **220,** dehydration cleavage product **222** is withdrawn from dehydration cleavage reaction zone **220** and added as a feed stream to distillation column **228** from which dehydration cleavage product overhead stream **224** and dehydration cleavage product bottoms stream **226** are withdrawn. Dehydration cleavage product overhead stream **224** is sent as at least a portion of feed stream **225** to hydrogenation reaction zone **230** along with hydrogen **231.** In some embodiments, the dehydration cleavage product bottoms stream **226** is sent as additional feed to dehydration cleavage reaction zone **220.** In some embodiments, a portion of stream **236** is added with stream **224** to form the feed stream **225** to the hydrogenation reaction zone **230** comprising a hydrogenation catalyst. After the reaction in hydrogenation reaction zone **230,** hydrogenation product **232** is withdrawn from hydrogenation reaction zone **230** and added as a feed stream to distillation column **238** from which hydrogenation product overhead stream **234** and hydrogenation product bottoms stream **236** are withdrawn. In some embodiments, the hydrogenation product bottoms stream **236** is sent as additional feed to dehydration cleavage reaction zone **220,** as additional feed to hydrogenation reaction zone **230,** or a combination thereof. In some embodiments, a portion of stream **236** is added with stream **224** to form the feed stream **225** to the hydrogenation reaction zone **230** comprising a hydrogenation catalyst.

As shown in **FIG. 2B****,** hydrogenation product overhead stream **234** is added as a feed stream to dehydration reaction zone **240.** After the reaction in dehydration reaction zone **240,** dehydration product **242** is withdrawn from dehydration reaction zone **240** and added as a feed stream to distillation column **248** from which dehydration product overhead stream **244** and dehydration product bottoms stream **246** are withdrawn.

In some embodiments, as shown in **FIG. 2C****,** an organic waste stream **202** containing impurities harmful to the dehydration cleavage catalyst is added as a feed stream **202** to guard reaction zone **210.** Guard reaction product **212** comprising on or more propylene glycols is withdrawn from guard reaction zone **210.**

### Certain Embodiments

In some embodiments, the process comprises adding a feed stream comprising one or more propylene polyols, and optionally water, to a dehydration cleavage reaction zone in the presence of a dehydration cleavage catalyst to form a first reaction mixture. In some embodiments, the one or more propylene polyols comprise propylene glycol, di-propylene glycol, tri-propylene glycol, tetra-propylene glycol, or a combination thereof.

The first reaction mixture is reacted at: a temperature in the range of from 20°C to 600°C, from 50°C to 500°C, or 100°C to 300°C; a pressure in the range of from 0 psig (0 kPag) to 1,000 psig (6,895 kPag), from 10 psig (68.9 kPag) to 300 psig (2,068 kPag), or from 50 psig (345 kPag) to 100 psig (689 kPag); or a combination thereof, to form a dehydration cleavage product stream comprising propionaldehyde, a dioxolane component, a dioxane component, 1-propanol, mono-propylene glycol, di-propylene glycol, and other oxygenates. In some embodiments, the dehydration cleavage product stream comprises water in the range of from 0 wt% to 90 wt%, wherein weight percentage water is based on the total weight of the dehydration cleavage product, and the propionaldehyde in the range of from 40 wt% to 80 wt%, the dioxolane component in the range of 5 wt% to 50 wt%, the dioxane component in the range of from 1 wt% to 20 wt%, 1-propanol in the range of from 0 wt% to 10 wt%, mono-propylene glycol in the range of from 0 wt% to 10 wt%, di-propylene glycol in the range of from 0 wt% to 10 wt%, (0-10%), and other oxygenates in the range of from 0 wt% to 30 wt%, wherein weight percentages are based on the total weight of the dehydration cleavage product stream other than water.

In some embodiments, the process further comprises either:
a) adding the dehydration cleavage stream and hydrogen to a hydrogenation reaction zone in the presence of a hydrogenation catalyst to form a second reaction mixture, wherein in some embodiments, the hydrogen is added in an amount such that the molar ratio of hydrogen (H₂) to oxygen (O) in the dehydration cleavage stream is in the range of from 1 to 20 or from 5 to 10; or
b) feeding the dehydration cleavage product to a first distillation column to produce a dehydration cleavage product overhead stream and a dehydration cleavage product bottoms stream, recovering the dehydration cleavage product overhead stream comprising propionaldehyde, a dioxolane component, and a dioxane component, and adding the dehydration cleavage product overhead stream and hydrogen to a hydrogenation reaction zone in the presence of a hydrogenation catalyst to form a second reaction mixture, wherein in some embodiments, the hydrogen is added in an amount such that the molar ratio of hydrogen (H₂) to oxygen (O) in the dehydration cleavage product overhead stream is in the range of from 1 to 20 or from 5 to 10.
In either instance, the second reaction mixture is reacted at: a temperature in the range of from 20°C to 600°C, from 50°C to 500°C, or from 100°C to 350°C; a pressure in the range of from 100 psig (689 kPag) to 1,500 psig (10,340 kPag), from 250 psig (1,724 kPag) to 1,250 psig (8,620), or from 500 psig (3,450 kPag) to 1,000 psig (6,890 kPag); or a combination thereof, to form a hydrogenation product stream comprising a propanol component. In some embodiments, the propanol component comprises n-propanol, iso-propanol, or a combination thereof. In some embodiments, the dehydration product stream further comprises a propylene precursor component, wherein in some cases the propylene precursor component comprises 1 propanol, 2-propanol, propionaldehyde, acetone, C₃ dioxanes, C₃ dioxolanes, propylene glycol, hydroxyacetone, or a combination thereof.

In some embodiments, the feed stream is added to the dehydration cleavage reaction zone at a weight hourly space velocity in the range of from 0.1 h⁻¹ to 100 h⁻¹, 0.5 h⁻¹ to 60 h⁻¹, or 0.8 h⁻¹ to 25 h⁻¹; the dehydration cleavage product stream and hydrogen are added to the hydrogenation reaction zone at a weight hourly space velocity in the range of from 0.1 h⁻¹ to 100 h⁻¹, 0.3 h⁻¹ to 50 h⁻¹, or 0.5 h⁻¹ to 5 h⁻¹; or any combination thereof.

In further embodiments:
a) at least a portion of the dehydration cleavage product or the dehydration cleavage product bottoms stream is added to the dehydration cleavage reaction zone as additional feed;
b) at least a portion of the hydrogenation product or the hydrogenation product bottoms stream is added to the dehydration cleavage reaction zone as additional feed, the hydrogenation reaction zone as additional feed, or a combination thereof; or
c) a combination thereof.

In some embodiments, in addition to any of the foregoing, the process further comprises either:
a) adding the hydrogenation product stream to a dehydration reaction zone in the presence of a dehydration catalyst to form a third reaction mixture; or
b) feeding the hydrogenation product to a second distillation column to produce a hydrogenation product overhead stream comprising a propanol component and a hydrogenation product bottoms stream, and adding the hydrogenation product overhead stream overhead stream to a dehydration reaction zone in the presence of a dehydration catalyst to form a third reaction mixture.

In either instance, the third reaction mixture is reacted at: a temperature in the range of from 20°C to 600°C, from 50°C to 400°C, or from 100°C to 300°C; a pressure in the range of from 15 psig (103 kPag) to 500 psig (689 kPag), from 100 psig (689 kPag) to 450 psig (3,100 kPag), from 200 psig (1,379 kPag) to 400 psig (2,760 kPag); or a combination thereof, to form a dehydration product comprising propylene. In some embodiments, the hydrogenation product stream or the hydrogenation product overhead stream is added to the dehydration reaction zone at a weight hourly space velocity in the range of from 0.1 h⁻¹ to 100 h⁻¹, 0.5 h⁻¹ to 60 h⁻¹, or 0.8 h⁻¹ to 25 h⁻¹.

In some embodiments, the dehydration product is fed to third distillation column to produce a dehydration product overhead stream comprising a propanol component and a dehydration product bottoms stream. In some embodiments, the dehydration product stream comprises propylene in the range of from 80 wt% to 100 wt% and other hydrocarbons in the range of from 0 wt% to 20 wt%, wherein weight percentages are based on the total weight of the dehydration product stream. In some embodiments, the other hydrocarbons comprise C₂, C₃, and/or C₄ oxygenates, C₂, C₃, and/or C₄ olefins, or a combination thereof.

In some embodiments, in addition to any one of the foregoing embodiments, the process further comprises adding an organic waste stream, comprising one or more propylene polyols and a first content of one or more impurities harmful to the dehydration cleavage catalyst, to a guard reaction zone to form the feed stream comprising one or more propylene polyols.

Any one of the foregoing embodiments can be further characterized in that the dehydration cleavage catalyst comprises a first solid acid catalyst. In some embodiments, the first solid acid catalyst component comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, one or more polystyrene sulfonate acidic resins, sulfonate functionalized support, tethered organic sulfonic acids, acidic clays, or a combination thereof. In some embodiments, the first solid acid catalyst is further characterized by one or more of the following:
a) the zeolite component comprises zeolite-H-Y, zeolite-H-ZSM5, zeolite-H-beta, zeolite-H-mordenite, zeolite-H-ferrierite, or a combination thereof;
b) the alumina silicate comprises amorphous alumina silicate, acid washed layered alumina silicate, or a combination thereof, wherein in some embodiments, the acid washed layered alumina silicate comprises bentonite, vermiculite, or a combination thereof;
d) the aluminum phosphate comprises SAPO 34;
e) the supported phosphoric acid comprises a silica, clay, or alumina support;
f) the one or more supported tungsten oxides comprise a silica, clay, or alumina support;
g) the one or more tungsten oxides comprise tungsten oxide, tungsten di-oxide, tungsten tri-oxide, tungsten pentoxide, or a combination thereof;
h) the supported tungstosilicic acid comprises a silica, clay, or alumina support;
i) the supported phosphomolybdic acid comprises a silica, clay, or alumina support; and
j) the sulfonate functionalized support is silica.

In some embodiments, the zeolite catalyst component of the first solid acid catalyst has a SiO₂/Al₂O₃ mole ratio of less than or equal to 200, less than or equal to 100, less than or equal to 50, less than or equal to 25, or less than or equal to 15. In some embodiments, the zeolite catalyst component has a SiO₂/Al₂O₃ mole ratio of greater than or equal to 0.5, greater than or equal to 1, greater than or equal to 3, greater than or equal to 5, or greater than or equal to 10. In some embodiments, the zeolite catalyst component has a SiO₂/Al₂O₃ mole ratio in the range of from 0.5 to 200, from 1 to 100, form 3 to 50, from 5 to 25, or from 10 to 15.

Any one of the foregoing embodiments can be further characterized in that the hydrogenation catalyst comprises nickel (Ni), Raney Ni, cobalt (Co), molybdenum (Mo), ceria (Ce), magnesium (Mg), gold (Au), iridium (Ir), osmium (Os), palladium (Pd), platinum (Pt), rhodium (Rh), ruthenium (Ru), tungsten (W), titanium (Ti), NiMo, CoMo, NiW, CoW, Ru, Pt, Pd, or a combination thereof. In some embodiments, the foregoing metal or metals are supported on silica, alumina, silica alumina, zeolite, activated carbon, or a combination thereof. In some embodiments, the catalyst is sulfided prior to hydrogenation. In some embodiments, the hydrogenation catalyst comprises sulfided NiMo/Al₂O₃, sulfided CoMo/Al₂O₃, Ni/SiO₂, Ni/Al₂O₃, Raney Ni, Cu/SiO₂, Cu/Al₂O₃, Pd/SiO₂, Pd/Al₂O₃, Pd/C, Pt/SiO₂, Pt/Al₂O₃, Ru/C, In₂O₃ In₂O₃/Al₂O₃, In₂O₃/SiO₂, or a combination thereof.

Any one of the foregoing embodiments can be further characterized in that the dehydration catalyst comprises a second solid acid catalyst. In some embodiments, the second solid acid catalyst comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, or a combination thereof. In some embodiments, the second solid acid catalyst is further characterized by one or more of the following:
a) the zeolite component comprises zeolite-H-Y, zeolite-H-ZSM5, zeolite-H-beta, zeolite-H-mordenite, zeolite-H-ferrierite, or a combination thereof;
b) the alumina silicate comprises amorphous alumina silicate, acid washed layered alumina silicate, or a combination thereof, wherein the acid washed layered alumina silicate comprises bentonite, vermiculite, or a combination thereof;
c) the aluminum phosphate comprises SAPO 34;
d) the supported phosphoric acid comprises a silica, clay, or alumina support;
e) the one or more supported tungsten oxides comprise a silica, clay, or alumina support; and
f) the one or more tungsten oxides comprise tungsten oxide, tungsten di-oxide, tungsten tri-oxide, tungsten pentoxide, or a combination thereof.

In some embodiments, the zeolite catalyst component of the second solid acid catalyst has a SiO₂/Al₂O₃ mole ratio of less than or equal to 200, less than or equal to 100, less than or equal to 50, less than or equal to 25, or less than or equal to 15. In some embodiments, the zeolite catalyst component has a SiO₂/Al₂O₃ mole ratio of greater than or equal to 0.5, greater than or equal to 1, greater than or equal to 3, greater than or equal to 5, or greater than or equal to 10. In some embodiments, the zeolite catalyst component has a SiO₂/Al₂O₃ mole ratio in the range of from 0.5 to 200, from 1 to 100, from 3 to 50, from 5 to 25, or from 10 to 15.

The presently disclosed methods for conversion of propylene polyols to propylene and/or propylene precursors are exemplified with respect to the examples below. These examples are included to demonstrate embodiments of the appended claims. However, these are exemplary only, and the disclosure can be broadly applied to any combination of propylene polyol feed, with and without water and/or impurities, and disclosed catalysts. In no way should the following examples be read to limit, or to define, the scope of the appended claims.

### EXAMPLES

The following examples are included to demonstrate embodiments of the disclosure. It should be appreciated by those of skill in the art that the techniques disclosed in the examples which follow represent techniques discovered to function well in the practice of the disclosure, and thus can be considered to constitute preferred modes for its practice.

### Raw Materials

In Examples 1-69, dehydration cleavage was demonstrated by reacting various propylene polyols ("PPOs") with various dehydration cleavage catalysts as shown in Table 1 below.

**TABLE 1**

| **Label** | **Catalyst Type** | **Grade** | **Available from** |
|---|---|---|---|
| AC1 | acidic clay | Bentonite F20XLM | Engineered Clay Corporation, Jackson, MS |
| AC2 | acidic clay | Bentonite F21 | Engineered Clay Corporation, Jackson, MS |
| AC3 | acidic clay | Bentonite F20X | Engineered Clay Corporation, Jackson, MS |
| AC4 | acidic clay | Bentonite Fulcat-22F | Southern Clay Products, Gonzalez, TX |
| AC5 | acidic clay | Bentonite Fulcat-435 | Southern Clay Products, Gonzalez, TX |
| Z1 | zeolite | Beta (CP 811 E 75) | Zeolyst, Valley Forge, PA |
| Z2 | zeolite | Ferrierite (Z870 TL 2.8) | Zeolyst, Valley Forge, PA |
| Z3 | zeolite | Ferrierite (ZD 18018 TL) | Zeolyst, Valley Forge, PA |
| Z4 | zeolite | H-USY (CFG-1) | Zeolyst, Valley Forge, PA |
| Z5 | zeolite | Mordenite | Zeolyst, Valley Forge, PA |
| Z6 | zeolite | TOSOH ZSM-5 | Tosoh, Grove City, OH |
| Z7 | zeolite | Y (CBV-720) | Zeolyst, Valley Forge, PA |
| Z8 | zeolite | ZSM-5 (CBV 3014) | Zeolyst, Valley Forge, PA |
| SA1 | sulfonic acid | Propyl-sulfonic acid/SiO₂ | Silicycle, Quebec City, Canada |
| SA2 | sulfonic acid | Tosic Acid/SiO₂ | Silicycle, Quebec City, Canada |
| TO1 ¹ | tungsten oxide | WO₃/SiO₂ | LyondellBasell Formulation |
| TO2 ¹ | tungsten oxide | WOₓ/SiO₂ | LyondellBasell Formulation |

| | | | |
|---|---|---|---|
| 1 Procedure to TO1 and TO2: 5.01g of spherical SiO₂ (AlphaCat 9640, PQ, Malvern, PA) was impregnated with a solution of 1.02g of ammonium metatungstate (SigmaAldrich, Milwaukee, WI) dissolved in 10ml of 30% H₂O₂ (SigmaAldrich, Milwaukee, WI). The resulting free-flowing particles were dried at 90oC for 5h the calcined at 350oC for 5h on air. | | | |

### Dehydration Cleavage Process

Dehydration cleavage reactions were performed in a custom built continuous packed bed reactor. The tube reactor consisted of a 6 inch (15.2 cm) long ¼" (6.4 mm) diameter stainless steel tube packed with 0.5 g of catalyst. The tube reactor was operated under isothermal conditions by placing the reactor inside an Agilent 7890 GC oven. Liquid feeds were fed using an ISCO 500D syringe pump to achieve the desired WHSV. Reactor pressure was set and maintained using a spring loaded back pressure regulator. Typical reaction pressures were 100 psig (689 kPag). Product compositions were analyzed by injecting the product stream on an Agilent 7890 GC equipped with a flame ionization detector (FID).

### Examples 1-28

Table 2 summarizes the results of Examples 1-28. Examples 1-28 were each performed using a sample of PPO continuously pumped over 0.5 g of an acidic clay as the solid acid catalyst. Respective columns in Table 2 show the conversion of PPO as a mole percentage the PPO in the feed. Subsequent columns of Table 2 show substituent components of the dehydration cleavage product other than water as a weight percentage of the total weight of the substituent components, in particular propionaldehyde, dioxolanes, dioxanes, DPG, MPG, acetone, hydroxyacetone, and n-propanol.

Table 2 further shows the type of PPO and the weight ratio of the PPO to water, if applicable and the WHSV of feed in the experimental dehydration cleavage reactor. All examples were performed at a pressure of 100 psig (689 kPag). All of Examples 1-28 show measurable PPO conversion but with a trend toward lower conversion at lower temperatures and/or higher WHSV.

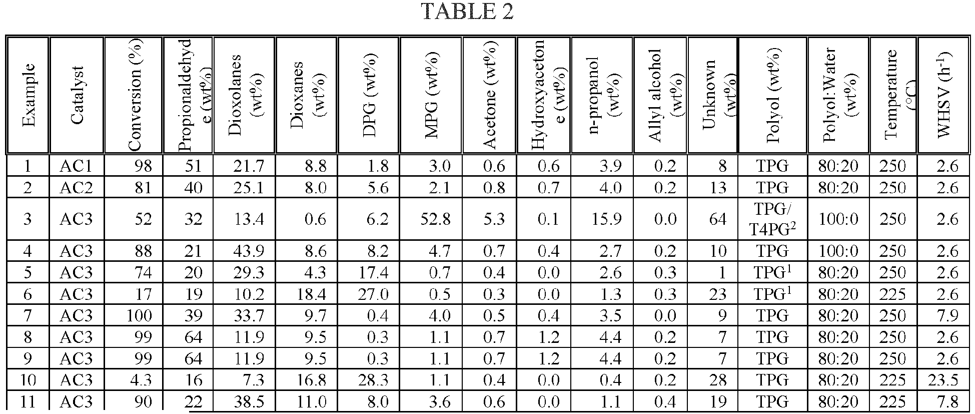

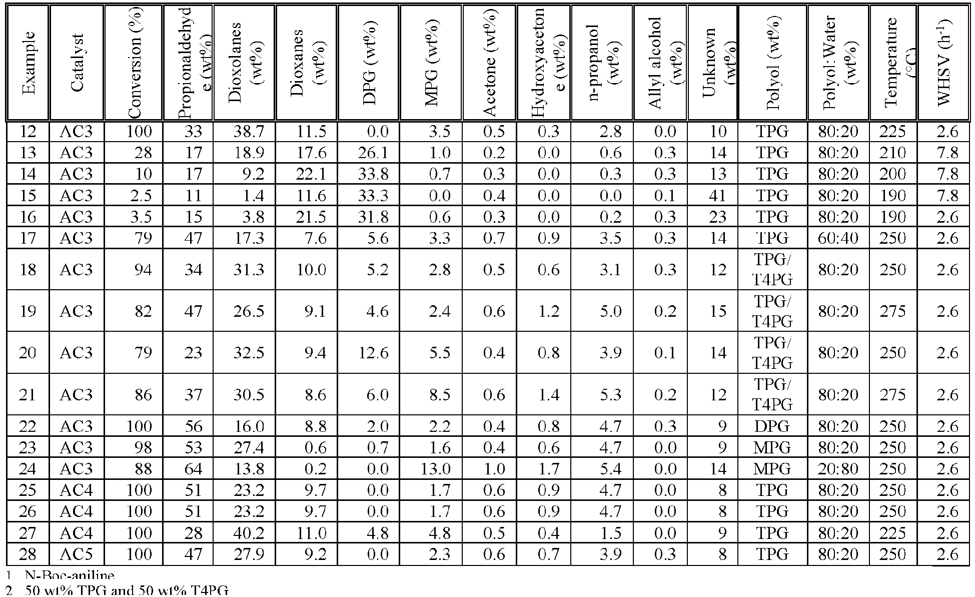

### Examples 29-65

Table 3 summarizes the results of Examples 29-65. Examples 29-65 were each performed using a sample of PPO continuously pumped over 0.5 g of a zeolite as the solid acid catalyst. Respective columns in Table 3 show the conversion of PPO as a mole percentage the PPO in the feed. Subsequent columns of Table 3 show substituent components of the dehydration cleavage product other than water as a weight percentage of the total weight of the substituent components, in particular propionaldehyde, dioxolanes, dioxanes, DPG, MPG, acetone, hydroxyacetone, and n-propanol.

Table 3 further shows the type of PPO and the weight ratio of the PPO to water, if applicable and the WHSV of feed in the experimental dehydration cleavage reactor. All examples were performed at a pressure of 100 psig (689 kPag). All of Examples 29-65 show measurable PPO conversion but with a trend toward lower conversion at lower temperatures and/or higher WHSV.

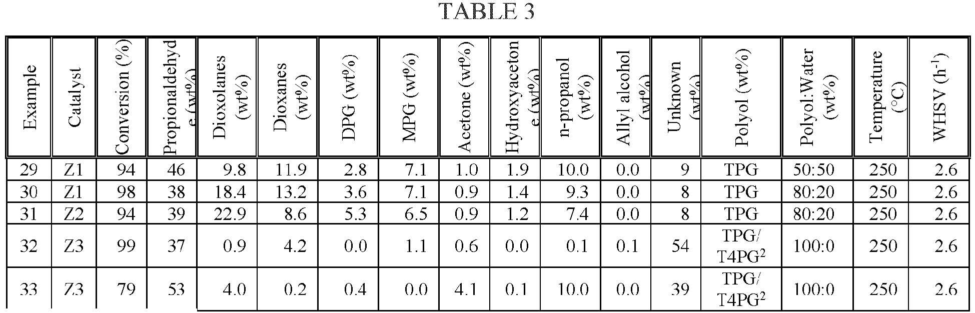

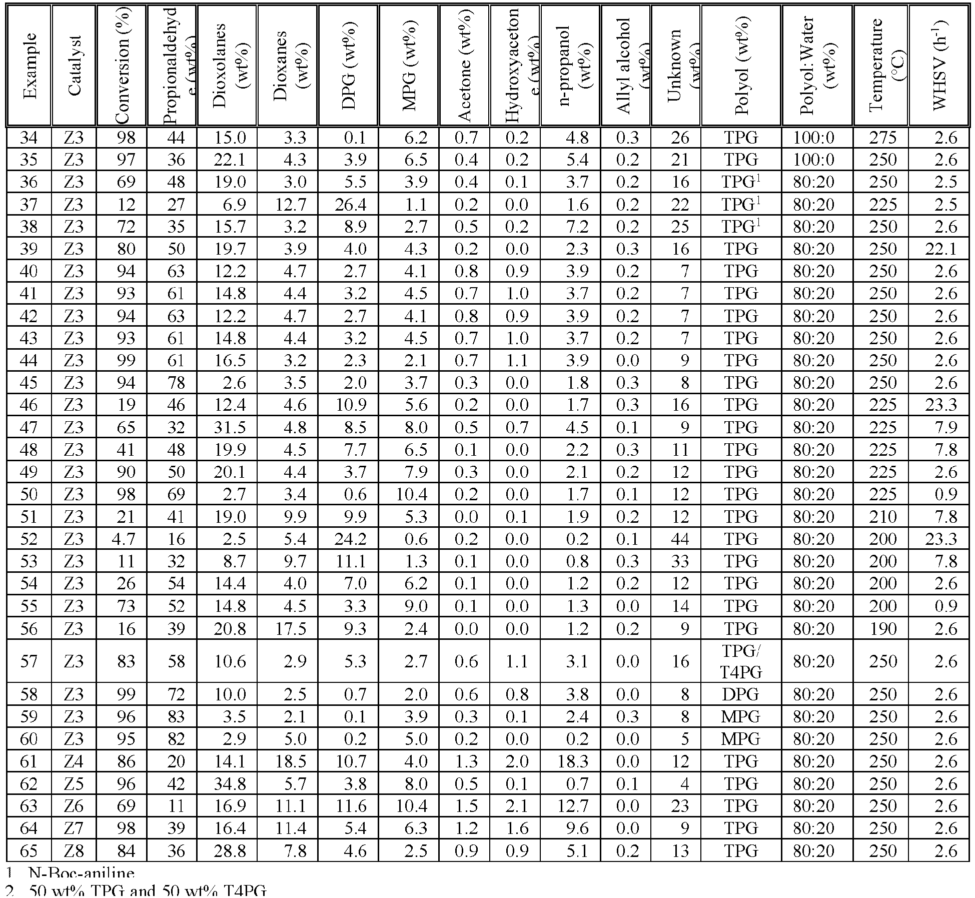

### Examples 66 and 67

Table 4 summarizes the results of Examples 66 and 67. Examples 66 and 67 were each performed using a sample of PPO continuously pumped over 0.5 g of a tethered organic acid as the solid acid catalyst. Respective columns in Table 4 show the conversion of PPO as a mole percentage the PPO in the feed. Subsequent columns of Table 4 show substituent components of the dehydration cleavage product other than water as a weight percentage of the total weight of the substituent components, in particular propionaldehyde, dioxolanes, dioxanes, DPG, MPG, acetone, hydroxyacetone, and n-propanol.

Table 4 further shows the type of PPO and the weight ratio of the PPO to water, if applicable and the WHSV of feed in the experimental dehydration cleavage reactor. All examples were performed at a pressure of 100 psig (689 kPag). Examples 66 and 67 show measurable PPO conversion.

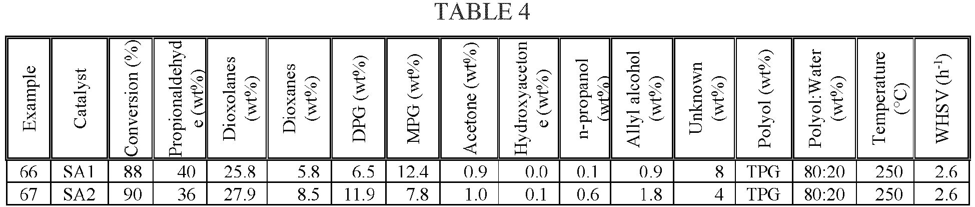

### Examples 68 and 69

Table 5 summarizes the results of Examples 68 and 69. Examples 68 and 69 were each performed using a sample of PPO continuously pumped over 0.5 g of a silica supported tungsten oxide catalyst as the solid acid catalyst. Respective columns in Table 5 show the conversion of PPO as a mole percentage the PPO in the feed. Subsequent columns of Table 5 show substituent components of the dehydration cleavage product other than water as a weight percentage of the total weight of the substituent components, in particular propionaldehyde, dioxolanes, dioxanes, DPG, MPG, acetone, hydroxyacetone, and n-propanol.

Table 5 further shows the type of PPO and the weight ratio of the PPO to water, if applicable and the WHSV of feed in the experimental dehydration cleavage reactor. All examples were performed at a pressure of 100 psig (689 kPag). Examples 68 and 69 show measurable PPO conversion.

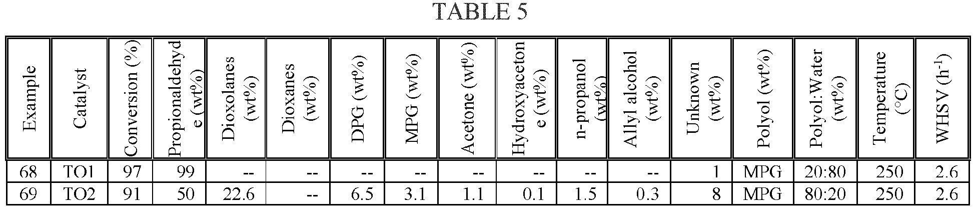

### Hydrogenation Process

Any product of Examples 1-69 and hydrogen can be fed to a hydrogenation reaction zone comprising NiMo (KL 8234, Shell) sulfided by Eurocat as a hydrogenation catalyst at a WHSV of 2.75 h⁻¹. Hydrogen can be added in an amount such that the molar ratio of hydrogen (H₂) to oxygen (O) in the dehydration cleavage product to the hydrogenation reaction zone is in the range of from 1 to 20 or from 5 to 10. A typical hydrogenation reaction can be conducted at a temperature of about 225°C and a pressure of 750 psig (5,170 kPag) to produce a hydrogenation product comprising 0 wt% to 90 wt% water and 10 wt% to 100 wt% organics other than water, wherein weight percentages are based on the total weight of the hydrogenation product. The organics other than water comprise 1-propanol in the range of from 50 wt% to 90 wt% and other C₃ hydrocarbons in the range of from 10 wt% to 50 wt%, wherein weight percentages are based on the total weight of the organics other than water.

### Dehydration Process

Any hydrogenation product produced from Examples 1-69 using the above hydrogenation reaction conditions can be fed to a dehydration reaction zone comprising aluminum oxide as a solid acid catalyst at a WHSV of about 1 to 10 h⁻¹. A typical dehydration reaction can be conducted at a temperature of about 200°C and a pressure of 300 psig (2,070 kPag) to produce a dehydration product comprising 80 wt% to 100 wt% propylene after removal of all water produced in the reaction.

For the sake of brevity, only certain ranges are explicitly disclosed herein. However, in addition to recited ranges, any lower limit may be combined with any upper limit to recite a range not explicitly recited, as well as, ranges from any lower limit may be combined with any other lower limit to recite a range not explicitly recited, in the same way, ranges from any upper limit may be combined with any other upper limit to recite a range not explicitly recited. Additionally, within a range includes every point or individual value between its end points even though not explicitly recited. Thus, every point or individual value may serve as its own lower or upper limit combined with any other point or individual value or any other lower or upper limit, to recite a range not explicitly recited.

Moreover, the scope of the present application is not intended to be limited to the particular embodiments of the processes, machines, means, methods, and/or steps described in the specification. As one of the ordinary skill in the art will readily appreciate from the present disclosure, processes, machines, means, methods, and/or steps, presently existing or later to be developed that perform substantially the same function or achieve substantially the same result as the corresponding embodiments described herein, may be utilized according to the present disclosure. Accordingly, the appended claims are intended to include within their scope such processes, machines, means, methods, and/or steps.

## Claims

1. A process comprising:
a) adding a feed stream comprising one or more propylene polyols and optionally water to a dehydration cleavage reaction zone in the presence of a first solid acid catalyst to form a first reaction mixture;
b) reacting the first reaction mixture at a temperature in the range of from 20°C to 600°C, a pressure in the range of from 0 psig (0 kPag) to 1,000 psig (6,895 kPag), or a combination thereof, to form a dehydration cleavage product stream comprising propionaldehyde, a dioxolane component, and a dioxane component;
c) adding the dehydration cleavage product stream and hydrogen to a hydrogenation reaction zone in the presence of a hydrogenation catalyst to form a second reaction mixture; and
d) reacting the second reaction mixture at a temperature in the range of from 20°C to 600°C, a pressure in the range of from 100 psig (689 kPag) to 1,500 psig (10,340 kPag), or a combination thereof, to form a hydrogenation product stream comprising a propanol component.

2. The process of claim 1, wherein the one or more propylene polyols comprise propylene glycol, di-propylene glycol, tri-propylene glycol, tetra-propylene glycol, or a combination thereof.

3. The process of claim 1, wherein the dehydration cleavage product stream comprises:
a) water in the range of from 0 wt% to 90 wt%, wherein weight percentage is based on the total weight of the dehydration cleavage product, and
b) the propionaldehyde in the range of from 40 wt% to 80 wt%, the dioxolane component in the range of 5 wt% to 50 wt%, and the dioxane component in the range of from 1 wt% to 20 wt%, wherein weight percentages are based on the total weight of the dehydration cleavage product stream other than water.

4. The process of claim 1, wherein the hydrogenation product comprises 0 wt% to 90 wt% water and 10 wt% to 100 wt% organics other than water, wherein weight percentages are based on the total weight of the hydrogenation product.

5. The process of claim 4, wherein the organics other than water comprise 1-propanol in the range of from 50 wt% to 90 wt% and other C₃ hydrocarbons in the range of from 10 wt% to 50 wt%, wherein weight percentages are based on the total weight of the organics other than water.

6. The process of claim 1, wherein the hydrogenation product stream further comprises a propylene precursor component.

7. The process of claim 1, further comprising:
a) adding at least a portion of the dehydration cleavage product to the dehydration cleavage reaction zone;
b) adding at least a portion of the hydrogenation product to the dehydration cleavage reaction zone. the hydrogenation reaction zone, or a combination thereof; or
c) a combination thereof.

8. The process of claim 1, wherein the first solid acid catalyst component comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, one or more polystyrene sulfonate acidic resins, sulfonate functionalized support, tethered organic sulfonic acids, acidic clays, or a combination thereof.

9. The process of claim 1, wherein the hydrogenation catalyst comprises sulfided NiMo/Al₂O₃, sulfided CoMo/Al₂O₃, Ni/SiO₂, Ni/Al₂O₃, Raney Ni, Cu/SiO₂, Cu/Al₂O₃, Pd/SiO₂, Pd/Al₂O₃, Pd/C, Pt/SiO₂, Pt/Al₂O₃, Ru/C, In₂O₃ In₂O₃/Al₂O₃, In₂O₃/SiO₂, or a combination thereof.

10. The process of claim 1, further comprising:
a) adding the hydrogenation product stream to a dehydration reaction zone in the presence of a second solid acid catalyst to form a third reaction mixture; and
b) reacting the third reaction mixture at a temperature in the range of from 20°C to 600°C, a pressure in the range of from 15 psig (103 kPag) to 500 psig (3447 kPag), or a combination thereof, to form a dehydration product stream comprising propylene.

11. The process of claim 10, wherein the second solid catalyst component comprises a zeolite component, an alumina silicate component, aluminum phosphate, zirconium sulfate, titanium sulfate, supported phosphoric acid, one or more supported tungsten oxides, supported tungstosilicic acid, supported phosphomolybdic acid, aluminum oxide, niobium oxide, or a combination thereof.

12. The process of claim 1, further comprising adding an organic waste stream, comprising one or more propylene polyols and a first content of one or more impurities harmful to the dehydration cleavage catalyst, to a guard reaction zone to form the feed stream comprising one or more propylene polyols.

13. The process according to claim 1 comprising:
a) adding a feed stream comprising one or more propylene polyols and optionally water to a dehydration cleavage reaction zone in the presence of a dehydration cleavage catalyst to form a first reaction mixture;
b) reacting the first reaction mixture at a temperature in the range of from 20°C to 600°C, a pressure in the range of from 0 psig (0 kPag) to 1,000 psig (6,895 kPag), or a combination thereof, to form a dehydration cleavage product stream comprising propionaldehyde, a dioxolane component, and a dioxane component;
c) withdrawing the dehydration cleavage product as a first hydrogenation feed or routing the dehydration cleavage product to a first distillation column to produce a dehydration cleavage product overhead stream and a dehydration cleavage product bottoms stream and withdrawing the dehydration cleavage overhead product as a second hydrogenation feed;
d) adding the first hydrogenation feed and hydrogen or the second hydrogenation feed and hydrogen to a hydrogenation reaction zone in the presence of a hydrogenation catalyst to form a second reaction mixture;
e) reacting the second reaction mixture at a temperature in the range of from 20°C to 600°C, a pressure in the range of from 100 psig (689 kPag) to 1,500 psig (10,340 kPag), or a combination thereof, to form a hydrogenation product stream comprising a propanol component; and
f) withdrawing the hydrogenation product as a first propanol-containing product or routing the hydrogenation product to a second distillation column to produce a hydrogenation product overhead stream and a hydrogenation product bottoms stream and withdrawing the hydrogenation overhead product as a second propanol-containing product;
wherein at least one of the second hydrogenation feed and the second propanol-containing product is formed during the process.

14. The process of claim 13, further comprising:
a) adding at least a portion of the first hydrogenation feed or the second hydrogenation feed to the dehydration cleavage reaction zone;
b) adding at least a portion of the first propanol-containing product of the second propanol-containing product to the dehydration cleavage reaction zone. the hydrogenation reaction zone, or a combination thereof; or
c) a combination thereof.

15. The process of claim 13, further comprising:
a) adding the first propanol-containing product or the second propanol-containing product to a dehydration reaction zone in the presence of a dehydration catalyst to form a third reaction mixture; and
b) reacting the third reaction mixture at a temperature in the range of from 20°C to 600°C, a pressure in the range of from 15 psig (103 kPag) to 500 psig (3447 kPag), or a combination thereof, to form a dehydration product stream comprising propylene.

## Patentansprüche

1. Ein Verfahren, das umfasst:
a) Zuführen eines Einsatzstroms, der ein oder mehrere Propylenpolyole und gegebenenfalls Wasser enthält, in eine Dehydratisierungs-Spaltreaktionszone in Gegenwart eines ersten festen Säurekatalysators, um ein erstes Reaktionsgemisch zu bilden;
b) Umsetzen des ersten Reaktionsgemisches bei einer Temperatur im Bereich von 20 °C bis 600 °C, einem Druck im Bereich von 0 psig (0 kPag) bis 1.000 psig (6.895 kPag) oder einer Kombination davon, um einen Dehydratisierungs-Spaltproduktstrom zu bilden, der Propionaldehyd, eine Dioxolan-Komponente und eine Dioxan-Komponente umfasst;
c) Zugeben des Dehydratisierungs-Spaltproduktstroms und von Wasserstoff in eine Hydrierungsreaktionszone in Gegenwart eines Hydrierungskatalysators, um ein zweites Reaktionsgemisch zu bilden; und
d) Reagierenlassen des zweiten Reaktionsgemisches bei einer Temperatur im Bereich von 20 °C bis 600 °C, einem Druck im Bereich von 100 psig (689 kPag) bis 1.500 psig (10.340 kPag) oder einer Kombination davon, um einen Hydrierungsproduktstrom zu bilden, der eine Propanolkomponente umfasst.

2. Verfahren nach Anspruch 1, wobei das eine oder die mehreren Propylenpolyole Propylenglykol, Dipropylenglykol, Tripropylenglykol, Tetrapropylenglykol oder eine Kombination davon umfassen.

3. Verfahren nach Anspruch1 , wobei der Dehydratisierungs-Spaltproduktstrom umfasst:
a) Wasser im Bereich von 0 Gew.-% bis 90 Gew.-%, wobei der Gewichtsprozentsatz auf das Gesamtgewicht des Dehydratisierungsspaltprodukts bezogen ist, und
b) Propionaldehyd im Bereich von 40 Gew.-% bis 80 Gew.-%, die Dioxolankomponente im Bereich von 5 Gew.-% bis 50 Gew.-% und die Dioxankomponente im Bereich von 1 Gew.-% bis 20 Gew.-%, wobei sich die Gewichtsprozente auf das Gesamtgewicht des Stroms des Dehydratisierungsspaltprodukts, ausgenommen Wasser, beziehen.

4. Verfahren nach Anspruch 1 , wobei das Hydrierungsprodukt 0 Gew.-% bis 90 Gew.-% Wasser und 10 Gew.-% bis 100 Gew.-% andere organische Bestandteile als Wasser umfasst, wobei die Gewichtsprozente auf das Gesamtgewicht des Hydrierungsprodukts bezogen sind.

5. Verfahren nach Anspruch 4, wobei die organischen Verbindungen, die kein Wasser sind, 1-Propanol im Bereich von 50 Gew.-% bis 90 Gew.-% und andere C₃-Kohlenwasserstoffe im Bereich von 10 Gew.-% bis 50 Gew.-% umfassen, wobei sich die Gewichtsprozente auf das Gesamtgewicht der organischen Verbindungen, die kein Wasser sind, beziehen.

6. Verfahren nach Anspruch 1 , wobei der Hydrierungsproduktstrom ferner eine Propylenvorläuferkomponente umfasst.

7. Verfahren nach Anspruch 1 , das ferner umfasst:
a) Zugabe mindestens eines Teils des Dehydratisierungsspaltprodukts in die Dehydratisierungs-Spaltreaktionszone;
b) Zugabe zumindest eines Teils des Hydrierungsprodukts in die Dehydratisierungs-Spaltreaktionszone, die Hydrierungsreaktionszone oder eine Kombination davon; oder
c) eine Kombination davon.

8. Verfahren nach Anspruch 1, wobei die erste feste Säurekatalysatorkomponente eine Zeolithkomponente, eine Aluminium-Silikat-Komponente, Aluminiumphosphat, Zirkoniumsulfat, Titansulfat, Phosphorsäure auf Träger, ein oder mehrere Wolframoxide auf Träger, Wolframsiliciumsäure auf Träger, Phosphomolybdänsäure auf Träger, Aluminiumoxid, Nioboxid, ein oder mehrere saure Polystyrolsulfonatharze, einen sulfonatfunktionalisierten Träger, gebundene organische Sulfonsäuren, saure Tonerden oder eine Kombination davon umfasst.

9. Verfahren nach Anspruch 1 , wobei der Hydrierungskatalysator sulfidiertes NiMo/Al₂O₃, sulfidiertes CoMo/Al₂O₃, Ni/SiO₂, Ni/Al₂O₃, Raney Ni, Cu/SiO₂, Cu/Al₂O₃ , Pd/SiO₂ , Pd/Al₂O₃ , Pd/C, Pt/SiO₂ , Pt/Al₂O₃ , Ru/C, In₂O₃ In₂O₃/Al₂O₃ , In₂O₃/SiO₂ oder eine Kombination davon.

10. Verfahren nach Anspruch 1, das ferner umfasst:
a) das Einleiten des Hydrierungsproduktstroms in eine Dehydratisierungsreaktionszone in Gegenwart eines zweiten festen Säurekatalysators, um ein drittes Reaktionsgemisch zu bilden; und
b) Umsetzen des dritten Reaktionsgemisches bei einer Temperatur im Bereich von 20 °C bis 600 °C, einem Druck im Bereich von 15 psig (103 kPag) bis 500 psig (3447 kPag) oder einer Kombination davon, um einen Dehydratisierungsproduktstrom zu bilden, der Propylen enthält.

11. Verfahren nach Anspruch 10 , wobei die zweite feste Katalysatorkomponente eine Zeolithkomponente, eine Aluminiumsilikatkomponente, -Aluminiumphosphat , Zirkoniumsulfat, Titansulfat, geträgerte Phosphorsäure, ein oder mehrere geträgerte Wolframoxide, geträgerte Wolfram-Kieselsäure, geträgerte Phosphomolybdänsäure, Aluminiumoxid, Nioboxid oder eine Kombination davon umfasst.

12. Verfahren nach Anspruch 1 , das ferner das Hinzufügen eines organischen Abfallstroms, der ein oder mehrere Propylenpolyole und einen ersten Gehalt an einer oder mehreren für den Dehydratisierungsspaltkatalysator schädlichen Verunreinigungen umfasst, zu einer Schutzreaktionszone umfasst, um den Einsatzstrom zu bilden, der ein oder mehrere Propylenpolyole umfasst.

13. Verfahren nach Anspruch 1, umfassend:
a) Zuführen eines Einsatzstroms, der ein oder mehrere Propylenpolyole und gegebenenfalls Wasser enthält, in eine Dehydratisierungs-Spaltreaktionszone in Gegenwart eines Dehydratisierungs-Spaltkatalysators, um ein erstes Reaktionsgemisch zu bilden;
b) Reagierenlassen des ersten Reaktionsgemisches bei einer Temperatur im Bereich von 20 °C bis 600 °C, einem Druck im Bereich von 0 psig (0 kPag) bis 1.000 psig (6.895 kPag) oder einer Kombination davon, um einen Dehydratisierungs-Spaltproduktstrom zu bilden, der Propionaldehyd, eine Dioxolan-Komponente und eine Dioxan-Komponente umfasst;
c) Abziehen des Dehydratisierungs-Spaltprodukts als ersten Hydrierungsbeschickungsstrom oder Leiten des Dehydratisierungs-Spaltprodukts zu einer ersten Destillationskolonne, um einen Dehydratisierungs-Spaltprodukt-Kopfstrom und einen Dehydratisierungs-Spaltprodukt-Bodenstrom zu erzeugen, und Abziehen des Dehydratisierungs-Spaltprodukt-Kopfstroms als zweiten Hydrierungsbeschickungsstrom;
d) Zugeben des ersten Hydrierungs-Einsatzmaterials und von Wasserstoff oder des zweiten Hydrierungs-Einsatzmaterials und von Wasserstoff in eine Hydrierungsreaktionszone in Gegenwart eines Hydrierungskatalysators, um ein zweites Reaktionsgemisch zu bilden;
e) Umsetzen des zweiten Reaktionsgemisches bei einer Temperatur im Bereich von 20 °C bis 600 °C, einem Druck im Bereich von 100 psig (689 kPag) bis 1.500 psig (10.340 kPag) oder einer Kombination davon, um einen Hydrierungsproduktstrom zu bilden, der eine Propanolkomponente umfasst; und
f) Entnahme des Hydrierungsprodukts als erstes propanolhaltiges Produkt oder Weiterleitung des Hydrierungsprodukts zu einer zweiten Destillationskolonne, um einen Hydrierungsprodukt-Kopfstrom und einen Hydrierungsprodukt-Bodenstrom zu erzeugen, und Entnahme des Hydrierungsprodukt-Kopfprodukts als zweites propanolhaltiges Produkt;
wobei während des Verfahrens mindestens entweder der zweite Hydrierungsbeschickungsstrom oder das zweite propanolhaltige Produkt gebildet wird.

14. Verfahren nach Anspruch 13 , das ferner umfasst:
a) Zugabe mindestens eines Teils des ersten Hydrierungs-Einsatzmaterials oder des zweiten Hydrierungs-Einsatzmaterials in die Dehydratisierungs-Spaltungsreaktionszone;
b) Zugabe mindestens eines Teils des ersten propanolhaltigen Produkts oder des zweiten propanolhaltigen Produkts in die Dehydratisierungs-Spaltungsreaktionszone, die Hydrierungsreaktionszone oder eine Kombination davon; oder
c) eine Kombination davon.

15. Verfahren nach Anspruch13 , das ferner umfasst:
a) Zugabe des ersten propanolhaltigen Produkts oder des zweiten propanolhaltigen Produkts zu einer Dehydratisierungsreaktionszone in Gegenwart eines Dehydratisierungskatalysators, um ein drittes Reaktionsgemisch zu bilden; und
b) Umsetzen des dritten Reaktionsgemisches bei einer Temperatur im Bereich von 20 °C bis 600 °C, einem Druck im Bereich von 15 psig (103 kPag) bis 500 psig (3447 kPag) oder einer Kombination davon, um einen Dehydratisierungsproduktstrom zu bilden, der Propylen enthält.

## Revendications

1. Procédé comprenant :
a) l'addition d'un flux d'alimentation comprenant un ou plusieurs polyols de propylène et, éventuellement, de l'eau, dans une zone de réaction de clivage par déshydratation en présence d'un premier catalyseur acide solide pour former un premier mélange réactionnel ;
b) la mise en réaction du premier mélange réactionnel à une température comprise entre 20 °C et 600 °C, sous une pression comprise entre 0 psig (0 kPag) et 1 000 psig (6 895 kPag), ou une combinaison de celles-ci, pour former un flux de produit de clivage par déshydratation comprenant de l'aldéhyde propionique, un composant dioxolane et un composant dioxane ;
c) l'addition d'un flux de produits de clivage par déshydratation et de l'hydrogène dans une zone de réaction d'hydrogénation en présence d'un catalyseur d'hydrogénation pour former un deuxième mélange réactionnel ; et
d) faire réagir le deuxième mélange réactionnel à une température comprise entre 20 °C et 600 °C, à une pression comprise entre 100 psig (689 kPag) et 1 500 psig (10 340 kPag), ou à une combinaison de celles-ci, pour former un flux de produit d'hydrogénation comprenant un composant propanol.

2. Procédé selon la revendication 1 , dans lequel le ou les polyols de propylène comprennent du propylène glycol, du dipropylène glycol, du tripropylène glycol, du tétrapropylène glycol, ou une combinaison de ceux-ci.

3. Procédé selon la revendication 1 , dans lequel le flux de produits de clivage par déshydratation comprend :
a) de l'eau dans une plage comprise entre 0 % et 90 % en poids, le pourcentage en poids étant basé sur le poids total du produit de clivage par déshydratation, et
b) du propionaldéhyde dans une plage comprise entre 40 % en poids et 80 % en poids, le composant dioxolane dans une plage comprise entre 5 % en poids et 50 % en poids, et le composant dioxane dans une plage comprise entre 1 % en poids et 20 % en poids, les pourcentages en poids étant basés sur le poids total du flux de produit de clivage par déshydratation autre que l'eau.

4. Procédé selon la revendication 1 , dans lequel le produit d'hydrogénation comprend de 0 % en poids à 90 % en poids d'eau et de 10 % en poids à 100 % en poids de composés organiques autres que l'eau, les pourcentages en poids étant basés sur le poids total du produit d'hydrogénation.

5. Procédé selon la revendication 4, dans lequel les composés organiques autres que l'eau comprennent du 1-propanol dans une plage de 50 % en poids à 90 % en poids et d'autres hydrocarbures en C₃ dans une plage de 10 % en poids à 50 % en poids, les pourcentages en poids étant basés sur le poids total des composés organiques autres que l'eau.

6. Procédé selon la revendication 1 , dans lequel le flux de produit d'hydrogénation comprend en outre un composant précurseur du propylène.

7. Procédé selon la revendication 1 , comprenant en outre :
a) l'ajout d'au moins une partie du produit de clivage par déshydratation dans la zone de réaction de clivage par déshydratation;
b) ajouter au moins une partie du produit d'hydrogénation à la zone de réaction de clivage par déshydratation, à la zone de réaction d'hydrogénation, ou à une combinaison de celles-ci ; ou
c) une combinaison de celles-ci.

8. Procédé selon la revendication 1 , dans lequel le premier composant catalyseur acide solide comprend un composant zéolitique, un composant silicate d'alumine, du phosphate d'aluminium, du sulfate de zirconium, de sulfate de titane, d'acide phosphorique supporté, d'un ou plusieurs oxydes de tungstène supportés, d'acide tungstosilicique supporté, d'acide phosphomolybdique supporté, d'oxyde d'aluminium, d'oxyde de niobium, d'une ou plusieurs résines acides de polystyrène sulfoné, d'un support fonctionnalisé par des groupes sulfonates, d'acides sulfoniques organiques liés, d'argiles acides, ou d'une combinaison de ceux-ci.

9. Procédé selon la revendication 1 , dans lequel le catalyseur d'hydrogénation comprend du NiMo/Al₂O₃ sulfuré, du CoMo/Al₂O₃ sulfuré, du Ni/SiO₂, du Ni/Al₂O₃, du Ni de Raney, du Cu/SiO₂, du Cu/Al₂O₃, du Pd/SiO₂, du Pd/Al₂O₃, du Pd/C, du Pt/SiO₂, du Pt/Al₂O₃, du Ru/C, de l'In₂O₃, de l'In₂O₃/Al₂O₃, de l'In₂O₃/SiO₂, ou une combinaison de ceux-ci.

10. Procédé selon la revendication 1, comprenant en outre :
a) l'ajout du flux de produit d'hydrogénation dans une zone de réaction de déshydratation en présence d'un deuxième catalyseur acide solide pour former un troisième mélange réactionnel ; et
b) la réaction du troisième mélange réactionnel à une température comprise entre 20 °C et 600 °C, sous une pression comprise entre 15 psig (103 kPag) et 500 psig (3447 kPag), ou une combinaison de celles-ci, pour former un flux de produit de déshydratation comprenant du propylène.

11. Procédé selon la revendication 10 , dans lequel le deuxième composant catalyseur solide comprend un composant zéolitique, un composant silicate d'alumine, du phosphate d'aluminium , du sulfate de zirconium, du sulfate de titane, de l'acide phosphorique supporté, un ou plusieurs oxydes de tungstène supportés, de l'acide tungstosilicique supporté, de l'acide phosphomolybdique supporté, de l'oxyde d'aluminium, de l'oxyde de niobium, ou une combinaison de ceux-ci.

12. Procédé selon la revendication 1 , comprenant en outre l'ajout d'un flux de déchets organiques, comprenant un ou plusieurs polyols de propylène et une première teneur en une ou plusieurs impuretés nocives pour le catalyseur de clivage par déshydratation, à une zone de réaction de protection afin de former le flux d'alimentation comprenant un ou plusieurs polyols de propylène.

13. Procédé selon la revendication 1, comprenant :
a) l'introduction d'un flux d'alimentation comprenant un ou plusieurs polyols de propylène et, éventuellement, de l'eau, dans une zone de réaction de clivage par déshydratation en présence d'un catalyseur de clivage par déshydratation afin de former un premier mélange réactionnel;
b) la mise en réaction du premier mélange réactionnel à une température comprise entre 20 °C et 600 °C, sous une pression comprise entre 0 psig (0 kPag) et 1 000 psig (6 895 kPag), ou une combinaison de celles-ci, pour former un flux de produit de clivage par déshydratation comprenant de l'aldéhyde propionique, un composant dioxolane et un composant dioxane;
c) retirer le produit de clivage par déshydratation en tant que première charge d'hydrogénation ou acheminer le produit de clivage par déshydratation vers une première colonne de distillation pour produire un flux de tête de produit de clivage par déshydratation et un flux de fond de produit de clivage par déshydratation, et retirer le produit de tête de clivage par déshydratation en tant que deuxième charge d'hydrogénation ;
d) ajouter la première charge d'hydrogénation et de l'hydrogène ou la deuxième charge d'hydrogénation et de l'hydrogène dans une zone de réaction d'hydrogénation en présence d'un catalyseur d'hydrogénation pour former un deuxième mélange réactionnel;
e) faire réagir le deuxième mélange réactionnel à une température comprise entre 20 °C et 600 °C, à une pression comprise entre 100 psig (689 kPag) et 1 500 psig (10 340 kPag), ou une combinaison de celles-ci, pour former un courant de produit d'hydrogénation comprenant un composant propanol; et
f) retirer le produit d'hydrogénation sous forme d'un premier produit contenant du propanol ou acheminer le produit d'hydrogénation vers une deuxième colonne de distillation pour produire un flux de tête de produit d'hydrogénation et un flux de fond de produit d'hydrogénation, et retirer le produit de tête d'hydrogénation sous forme d'un deuxième produit contenant du propanol;
dans lequel au moins l'une parmi la deuxième charge d'hydrogénation et le deuxième produit contenant du propanol est formée au cours du procédé.

14. Procédé selon la revendication 13 , comprenant en outre :
a) l'ajout d'au moins une partie de la première charge d'hydrogénation ou de la deuxième charge d'hydrogénation à la zone de réaction de clivage par déshydratation ;
b) l'ajout d'au moins une partie du premier produit contenant du propanol ou du deuxième produit contenant du propanol à la zone de réaction de clivage par déshydratation, à la zone de réaction d'hydrogénation, ou à une combinaison de celles-ci; ou
c) une combinaison de celles-ci.

15. Procédé selon la revendication 13 , comprenant en outre :
a) l'ajout du premier produit contenant du propanol ou du deuxième produit contenant du propanol dans une zone de réaction de déshydratation en présence d'un catalyseur de déshydratation pour former un troisième mélange réactionnel; et
b) la mise en réaction du troisième mélange réactionnel à une température comprise entre 20 °C et 600 °C, à une pression comprise entre 15 psig (103 kPag) et 500 psig (3447 kPag), ou une combinaison de celles-ci, pour former un flux de produit de déshydratation comprenant du propylène.
